# EUROPEAN PATENT APPLICATION

(11) **EP 2 829 256 A1**
(43) Date of publication of application: **28.01.2015**
(21) Application number: 13177874.8
(22) Date of filing: 24.07.2013
(51) Int. Cl.: A61F 13/15, A61F 13/47

(54) **Sanitary towel**

(71) Applicant: GG Innovation S.r.L., 59100 Prato (PO) (IT)
(72) Inventor: Govoni, Gabriella, I - 50127 FIRENZE (IT)
(74) Representative: Lunati & Mazzoni S.r.L.

(57) **Abstract**

A sanitary towel (1) is provided, comprising a contact layer (3) with the skin permeable to the passage of fluids; an outer layer (4) impermeable to the passage of fluids; a pad (2) suitable to absorb the fluids, and said layers (3, 4) are reciprocally joined by high frequency bonding forming an inner chamber (1 a) for the pad (2).

## Description

The present invention relates to a sanitary towel of the type as recited in the preamble of the first claim.

In particular, the invention relates to a sanitary towel, nappy, nursing pad bra liner, or other similar element suitable to be used to absorb liquids produced by the body preventing them from coming into contact with clothes, thereby staining them.

As known, sanitary towels are usually of the disposable type and have a flattened shape so as to be placed between the underwear and skin hindering movement and thereby determining a low level of comfort for the user.

The known sanitary towels are usually composed of a series of layers reciprocally joined by seams. In detail, they may be identified in an upper layer suitable to come into contact with the skin and to permit the transit of liquids, an absorbent layer suitable to absorb the liquids filtering through the upper layer, and a lower layer suitable to come into contact with the garments such as a bra or pair of knickers and prevent the passage of liquids, thereby preventing staining of said garment.

The prior art described above has several significant drawbacks.

A first significant drawback is the fact that ladies' sanitary towels, nappies or any other sanitary towel are disposable. They cannot therefore be re-utilised and must be thrown away every time.

Such drawback makes for elevated costs for the user, forced to purchase a conspicuous number of sanitary towels.

Moreover, the purchase of a conspicuous number of sanitary towels has a significant environmental impact.

In particular, such impact is determined both by the complex and expensive disposal methods and by the fact that to produce a disposable, sanitary towel, large amounts of raw materials are used (wood, plastic, water etc.) as well as particularly expensive and complex chemical processes.

To try to overcome these drawbacks, washable and recyclable sanitary towels have recently been introduced on the market.

Such sanitary towels are made practically entirely from natural, highly biodegradable fibre (usually starch fibre or cotton) permitting washing and thereby re-utilisation of the sanitary towel.

Such sanitary towels, while being a significant improvement compared to disposable sanitary towels, have several significant drawbacks.

A first significant drawback of such washable sanitary towels is the fact that already after the first washes, they rapidly deteriorate and are consequently no longer able to guarantee optimal absorption and, above all, sufficient capacity to retain liquids.

Such drawback is particularly evident at the seams, where on account of the stress suffered by the seams and natural fibres during washing, splits/apertures are formed through which an undesirable exit of the fluid from the sanitary towel occurs.

Another significant drawback, typical especially of sanitary towels made from starch fibre is the fact that such fibre breaks up already during the first washes and thereby rapidly degenerates.

As a result, even these sanitary towels, while being washable and recyclable, since they guarantee optimal absorption only for a limited number of uses, only partially resolve the problems associated with disposable sanitary towels.

In this situation the technical purpose of the present invention is to devise a sanitary towel able to substantially overcome the drawbacks mentioned above. Within the sphere of said technical purpose one important aim of the invention is to obtain a sanitary towel which can be re-used and, in particular, which is characterised by a capacity to maintain its absorption and liquid retention properties unaltered for a high number of uses.

Another important aim of the invention is to have a sanitary towel which permits a reduction in the costs of use and disposal.

A further aim of the invention is to devise a sanitary towel which is simple and practical to use.

The technical purpose and specified aims are achieved by a sanitary towel as claimed in the appended Claim 1.

Preferred embodiments are described in the dependent claims.

The characteristics and advantages of the invention are clearly evident from the following detailed description of a preferred embodiment thereof, with reference to the accompanying drawings, in which:
**Fig. 1** shows a sanitary towel according to the invention; and
**Fig. 2** shows a method of making the sanitary towel.

With reference to said drawings, reference numeral **1** globally denotes the sanitary towel according to the invention.

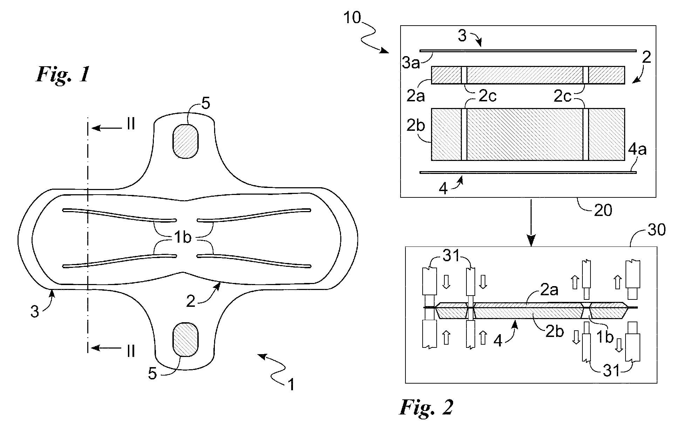

It is suitable for being placed between a garment and the body so as to absorb urine, milk, blood, faeces or other fluids produced by the body preventing them from coming into contact with garments and thereby staining them. The sanitary towel 1 can therefore be identified as a sanitary towel, (Fig. 1), a nappy, nursing pad bra liner.

The sanitary towel 1 comprises, mainly, a pad **2** suitable to absorb fluids; a contact layer **3** with the skin permeable to the passage of fluids; and an outer layer **4** suitable to come into contact with a garment and to be joined to the contact layer 3 defining an inner chamber **1a** for the pad 2 and, appropriately, one or more flow channels **1b,** preferably four, suitable to direct the fluid to the central part of the pad 2.

The contact layer 3 is made from an elastic textile comprising elastam. In particular, it is made from a textile made on weaving machines with a ladder-resist type loom, and composed of an elastic yarn, preferably elastam, and an inelastic yarn , appropriately polyamide microfibre.

Preferably, the contact layer textile 3 is of the antibacterial type obtained using, as the inelastic yarn, a natural fibre with intrinsic anti bacterial properties and/or treating the textile of the layer 3 with an antibacterial finishing process.

The outer layer 4 is of the type impermeable to the passage of fluid so as to prevent the fluid from reaching the garment and thereby staining it.

Preferably, the outer layer 4 is made from polyester.

Both layers 3 and 4 further comprise coatings **3a** and **4a** suitable to come into reciprocal contact and to substantially merge forming the high frequency bonding between the layers 3 and 4.

These coatings 3a and 4a are in polyvinyl or polyurethane or other polymer material. In detail, such coatings 3a and 4a are in polyurethane and, more specifically in polyurethane and polyamide.

Appropriately placed between the layers 3 and 4, the sanitary towel 1 has the pad 2.

The pad 2 comprises an acquisition distribution layer (ADL) **2a** and an absorbent body **2b** suitable to absorb the liquid.

The acquisition and distribution layer 2a is suitable to receive the fluid through the contact layer 3 and to favour its distribution along the surface of the sanitary towel 1, and specifically, of the absorbent body 2b. To such purpose, it comprises a porous thickness suitable to guarantee a rapid acquisition of fluid; and a distribution thickness consisting of a capillary structure suitable to define a plurality of directions of flow of the fluid and thus favour the distribution of the fluid along an extensive surface of the absorbent body 2b.

The absorbent body 2b is suitable to absorb and retain the liquids absorbed by the sanitary towel 1.

It comprises a felted mat of fibres, preferably polyester, comprising deep grooves/channels made along the longitudinal axis of said fibres so as to ensure a high level of absorption by the absorbent body. In particular, the fibres constituting the absorbent body 2b have a plurality of grooves and consequently, a multi-lobal cross-section. More in particular, said fibres are at least pentalobal and more in particular are even octalobal.

Preferably, the absorbent body 2b is made from 4dg^{®}, marketed by Fiber Innovation Technology.

Additionally, the absorbent body may comprise, in addition to the aforesaid mat, an antibacterial and preferably bacteriostatic fibre and a further mat in polyester fibre with a cross-shaped cross-section.

The antibacterial fibre is suitably made from polymer material with silver particles added and even more specifically from a fibre commercially known as Trevira Bioactive®.

Preferably, the absorbent body 2b comprises multilobal fibre and fibres with a cross-shaped cross-section in percentages substantially comprised between 30% and 50% and the antibacterial fibre in a percentage substantially equal to 20%.

Alternatively, the absorbent body 2b is made from a super absorbent polyester mat.

Additionally, the pad 2 comprises an additional absorbent body, not shown in the drawings, placed between the body 2b and outer layer 4 and suitable to increase the absorption capacity of the sanitary towel 1.

Said additional absorbent body is made in one of the materials described above for the absorbent body 2b.

Lastly, the sanitary towel 1 may have connection means **5,** such as velcro, suitable for joining the sanitary towel 1 to a garment (such as a pair of knickers or bra) so as to prevent unwanted relative shifting between the sanitary towel 1 and the garment.

The functioning of a sanitary towel, described above in a structural sense, is as follows.

After connecting the sanitary towel 1 to the garment, positioning the outer layer 4 in contact with said garment, the user wears the garment, placing the sanitary towel 1 and in particular the layer 3 in contact with the body.

During a discharge of fluid, it initially comes into contact with the layer 3 which, being permeable, allows the fluid to pass through it.

The fluid thus reaches the distribution and acquisition layer 2a which, thanks to its structure, rapidly absorbs the fluid, and contemporarily distributes it along its length.

The fluid lastly reaches the absorbent body 2b which absorbs and retains it, preventing its exit.

When the user decides to replace the sanitary towel 1, she/he detaches it from the garment, washes it in a washing machine and dries it for further use.

The invention comprises a new production method **10** of making sanitary towels 1.

The production method 10 comprises, mainly, an assembly phase **20** in which the various components of the sanitary towel 1 after being prepared, are laid over each other; and a bonding step 30 in which the layers 3 and 4 are reciprocally joined by high frequency bonding forming an inner chamber 1a for the pad 2 and preferably, flow channels 1b.

In detail, in the assembly step 20 the operator initially cuts the contact 3 and outer 4 layers, respectively from permeable, elastic and antibacterial sheets of fabric and from polyester sheets and deposits on each of these a coating 3a or 4a.

He then prepares the acquisition and distribution layer 2a and the absorbent body 2b, cutting through recesses **2c** into them, substantially counter-shaped to the flow channels 1b.

At this point the operator terminates the assembly step 20 placing the pad 2 between the contact layer 3 and the outer layers 4 and, in particular, placing the acquisition and distribution layer 2a between the absorbent body 2b and the contact layer 3.

The bonding step 30 then commences in which the layers 3 and 4 and, precisely, the coatings 3a and 4a are at least partially brought into reciprocal contact and then, connected to each other by high frequency bonding.

First of all, one or more bonding heads 31 reciprocally draw together the portions of the layers 3 and 4 not in contact with the pad 2, that is, the portions of the layers 3 and 4 external to the pad 2 and positioned in correspondence with the recesses 2c.

Then, the heads 31 apply to such portions of the layers 3 and 4 a clamping pressure substantially ranging from 100-150 bar, preferably practically ranging from 110-130 bar and even more preferably substantially equal to 120 bar. Almost contemporarily, the bonding heads 31 perform the high frequency bonding between such portions of the layers 3 and 4 applying a bonding frequency substantially ranging from 20-30 MHz, in particular substantially ranging from 25-30 MHz and, more in particular, substantially equal to 27 MHz. Such action of the bonding heads 31 heats and consequently melts almost exclusively the coatings 3a and 4a which thereby form the bonding, while the remaining parts of the layers 3 and 4 are practically not heated and thus do not substantially vary in their temperature or, as a result, in their structure.

The invention achieves some important advantages.

A first important advantage lies in the fact that the sanitary towel 1, unlike the nappies and sanitary towels of the prior art, is easy to wash and can therefore be re-utilised a number of times.

Such aspect is innovatively achieved thanks to the use of high frequency bonding which by causing heating of the coatings 3a and 4a only, does not alter the characteristics of the sanitary towel 1, and in particular of the layers 3 and 4.

Another advantage lies in the fact that the high frequency bonding forms particularly resistant bonds, and above all which do not deteriorate during washing as happens with seams or other solutions used in the sanitary towels of the prior art.

One important advantage therefore lies in the fact that, the sanitary towel 1 does not deteriorate during washing and consequently can be used a large number of times.

Such aspect is further increased by the particular materials used to make the sanitary towel 1 which, unlike starch fibre, does not break up during washing and, consequently, maintains its physical-chemical characteristics unaltered.

In fact, as shown by tests performed by the applicant, the sanitary towel 1 is able to withstand multiple washes in the washing machine at 30°C with spinning at 600 revs/minutes without detracting from its ability to absorb and retain liquids.

Another advantage is given by the presence of the connection means 5 making it possible to attach or detach the sanitary towel 1 quickly and easily from any garment.

For example, by means of the connection means 5, the operator can very easily attach the sanitary towel 1 to a pair of knickers, use such assembly as a normal sanitary towel and, once used, detach the sanitary towel 1 from the pair of knickers, wash the sanitary towel 1 and lastly reattach it to the knickers forming a new sanitary towel-knicker assembly.

A further advantage is given by the use of an acquisition and distribution layer 2a which, by ensuring particularly rapid absorption, allows the contact layer 3 to dry rapidly and remain in such state.

Such aspect is also achieved thanks to the use of the absorbent body 2b which guarantees, as well as a high absorption capacity, an optimal retention of the fluid absorbed.

Modifications and variations may be made to the invention described herein without departing from the scope of the inventive concept. All the elements as described and claimed herein may be replaced with equivalent elements and the scope of the invention includes all other details, materials, shapes and dimensions.

## Claims

1. Sanitary towel (1) comprising a pad (2) suitable to absorb fluids; a contact layer (3) with the skin permeable to the passage of said fluids; an outer layer (4) impermeable to the passage of said fluids; and **characterised in that** said contact layer (3) and said outer layer (4) are reciprocally joined by high frequency bonding forming an inner chamber (1a) for said pad (2).

2. Sanitary towel (1) as claimed in claim 1, in which said layers (3, 4) comprise coatings (3a, 4a); and in which said high frequency bonding between said layers (3, 4) is performed substantially by melting said coatings (3a, 4a).

3. Sanitary towel (1) as claimed in the previous claim, in which said coatings (3a, 4a) are in polyurethane.

4. Sanitary towel (1) as claimed in one or more of the previous claims, in which said contact layer (3) is made from an elastic textile.

5. Sanitary towel (1) as claimed in the previous claim, in which said contact layer (3) is made from an elastic textile of the ladder-proof type.

6. Sanitary towel (1) as claimed in one or more of the claims 4-5, in which the contact layer (3) is of the antibacterial type.

7. Sanitary towel (1) as claimed in one or more of the previous claims, in which said pad (2) comprises an acquisition and distribution layer (2a).

8. Sanitary towel (1) as claimed in one or more of the previous claims, in which said pad (2) comprises an absorbent body (2b) suitable to absorb the liquid and comprising an octalobal fibre mat.

9. Production method (10) of a sanitary towel (1) comprising a pad (2) suitable to absorb fluids, a contact layer (3) with the skin permeable to the passage of said fluids and an outer layer (4) impermeable to the passage of said fluids; said production method (10) being **characterised in that** it comprises a bonding step (30) in which the layers 3 and 4 are reciprocally joined by high frequency bonding forming an inner chamber (1a) for said pad (2).

10. Production method (10) as claimed in the previous claim, in which said high frequency bonding is performed at a bonding frequency substantially equal to 27 MHz.
